(19) Europäisches Patentamt / European Patent Office / Office européen des brevets

(11) **EP 4 106 734 B1**

(12) **EUROPEAN PATENT SPECIFICATION**

(45) Date of publication and mention
of the grant of the patent:
**07.06.2023   Bulletin 2023/23**

(21) Application number: **21719642.7**

(22) Date of filing: **20.04.2021**

(51) International Patent Classification (IPC):
*A61K 9/48* *(2006.01)*     *A61K 47/02* *(2006.01)*
*A61K 9/00* *(2006.01)*

(52) Cooperative Patent Classification (CPC):
**A61K 9/0075; A61K 9/4816; A61K 47/02**

(86) International application number:
**PCT/EP2021/060220**

(87) International publication number:
**WO 2021/214045 (28.10.2021 Gazette 2021/43)**

(54) **HPMC CAPSULE WITH REDUCED POWDER RETENTION**

HPMC-KAPSEL MIT REDUZIERTER PULVERRÜCKHALTUNG

CAPSULE HPMC À RÉTENTION DE POUDRE RÉDUITE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **22.04.2020   US 202063013917 P
04.05.2020   EP 20172719
04.02.2021   EP 21155241
25.02.2021   EP 21159324
26.02.2021   EP 21159631**

(43) Date of publication of application:
**28.12.2022   Bulletin 2022/52**

(73) Proprietor: **Capsugel Belgium NV
2880 Bornem (BE)**

(72) Inventors:
• **STAKA, Ivana**
**68000 Colmar (FR)**
• **CAPE, Jonathan**
**Bend, Oregon 97702 (US)**
• **PALANGETIC, Ljiljana**
**68000 Colmar (FR)**
• **VANQUICKENBORNE, Stefaan**
**2820 Rijmenam (BE)**

(74) Representative: **De Clercq & Partners
Edgard Gevaertdreef 10a
9830 Sint-Martens-Latem (BE)**

(56) References cited:
**US-A1- 2017 087 092     US-A1- 2019 321 301**

**Description**

The invention discloses hydroxypropyl methylcellulose (HPMC) capsules containing a small amount of $CaCl_2$, which show reduced powder retention when used in Dry Powder Inhalers (DPI), and a method for making them.

**BACKGROUND OF THE INVENTION**

Capsules are used in the pharmaceutical industry to allow oral administration of drugs or to administer powder for inhalation. In the latter case, the capsule is pierced using an adequate inhalation device and the powder is inhaled via the mouth or sometimes via the nose.

US 2015/0231344 A1 discloses a dry powder inhaler system comprising a HPMC capsule filled with a powder formulation containing a micronized active ingredient and a single dose dry powder inhaler device especially adapted to said capsule. [0011] mentions that some amount of drug will be still present in the capsule after inhalation.

US 5,626,871 discloses capsules for use in intratracheobronchial administration of powder contain in these capsules. The capsules are composed of HPMC, the gelling agent carrageenan and the gelling aid $K^+$, added as KCl, as disclosed in Example 17.

HPMC is a polymer that gels vice versa compared to a conventional film forming polymer such as gelatin: It can be completely dissolved at ambient like temperatures and gels only at elevated temperatures, whereas gelatin gels at ambient like temperatures and is dissolved at elevated temperatures. HPMC capsules, which are produced using the conventional gelling technique, that is at ambient like temperatures, require a gelling agent in order to gel at such ambient like temperatures.

Typical gelling agents are known to the skilled person and may be gellan gum, carrageenan, konjac gum, xanthan gum, and guar gum and the like.

Gelling agents are often used in combination with a gelling aid, which is usually a cation such as potassium or calcium. The gelling aid enhances the gelling ability of the gelling agent.

CN 189 846 851 A discloses such HPMC capsules which are prepared with the help of a gelling agent using the conventional gelling technique. The gelling agent is gellan gum in Examples 1, 2 and 3 and konjac gum in Example 4, 5 and 6. Further gelling agents are mentioned in the description in [0012].

The cations in form of their salts, which are disclosed in the examples, such as potassium and specifically calcium salts, serve as gelling aids.

CN 106 166 143 B discloses such HPMC capsules which are prepared with the help of a gelling agent using the conventional gelling technique. Gelling agents mentioned in the examples are gellan gum, carrageenan and pectin.

The cations in form of their salts, which are disclosed in the examples, such as potassium and specifically calcium salts, serve as coagulant, that is as gelling aids.

US 5 626 871 A discloses in Examples 17-21 such HPMC capsules which have been prepared with the help of a gelling agent using the conventional gelling technique. The gelling agent mentioned is carrageenan.

The potassium in form of its salt potassium chloride, which is disclosed in the examples 17-21, serves as gelling aid.

US 2019/0321301 A1 discloses acid resistant capsules comprising pectin and a divalent cation such as calcium-chloride in the capsule shells.

US 2017/0087092 A1 discloses a high-performance manufacturing method for hard capsule shells prepared with HPMC2906, but no calcium-chloride.

None of the above cited documents, whether alone or in combination, gives any motivation to change for DPI applications from HPMC capsules containing a gelling agent to gelling agent free HPMC capsule, even less they give a motivation to use small amounts of $CaCl_2$ in gelling agent free HPMC capsules.

A gelling agent may have a detrimental effect on the performance of the capsule, for example the presence of a gelling agent in the HPMC capsules may interfere with components in the dissolution media such as ions, specifically cations, or else, leading to changed and to different dissolution profiles. This is not desired.

There is a second method to gel HPMC, which is thermal gelation method, the HPMC is gelled at elevated temperature above the gelling point of HPMC. In the thermal gelation method no gelling agent is required.

It was found that pure HPMC capsules show less favorable powder retention properties compared with HPMC capsules containing a gelling agent.

There was a need for a HPMC capsule which does not contain any gelling agent while showing at least comparable average powder retention properties compared to HPMC capsules containing a gelling agent, in order to exclude any differences in the dissolution properties caused by the interaction of any ions or else in the dissolution media with a gelling agent. Furthermore the mechanical properties need to be acceptable for the production of capsules and for their use.

Also parameters which are important for the functioning of the capsules in the intended use, that is in a DPI apparatus, should be met, such as the puncturing and opening of the capsules should function according to the needs. So for example the puncturing force needs to provide for an efficient opening of the capsules without or at least with minimum fragmenting.

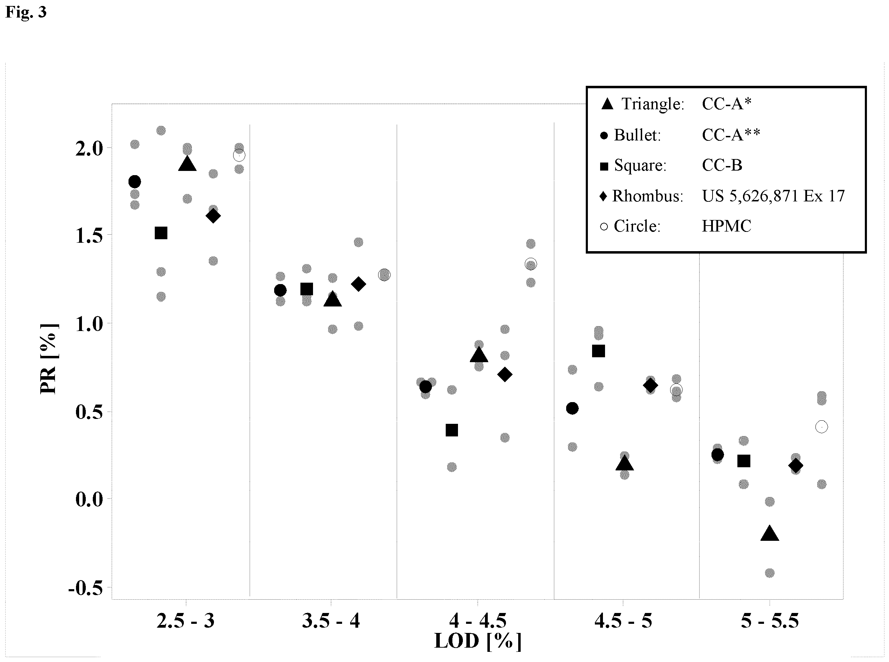

Surprisingly the problem was solved by adding a certain amount of CaCh into the shell material of the HPMC capsule in the absence of a gelling agent. The average powder retention properties as shown in Example 4 and displayed in Figure 3 are better than those of pure HPMC capsules, and they are at least comparable, under some circumstances even better compared to HPMC capsules containing a gelling agent. The mechanical properties in the impact test as shown in Example 3 are equivalent to pure HPMC capsules. The puncture test as shown in Example 5 shows even better performance than HPMC capsules containing a gelling agent.

**Abbreviations and definitions used in this specification**

| API | active pharmaceutical ingredient |
|---|---|
| cP | centipoise, it is one hundredth of a poise, or one millipascal-second (mPa·s) in SI units (1 cP = $10^{-3}$ Pa·s = 1 mPa·s) |
| DPI | Dry Powder Inhaler |
| HPMC | Hydroxypropyl methylcellulose, also called Hypromellose, Cellulose, 2-hydroxypropyl methyl ether; Cellulose hydroxypropyl methyl ether, [9004-65-3]. |
| | The definitions of Hypromellose which are used in instant invention can be found in: |
| | US Pharmacopeia |
| | Document Type: USP & NF |
| | DocId: 1_GUID-6A0B0F3C-FA70-433C-AD55-2020BBC64718_4_en-US Printed from: https://online.uspnf.com/uspnf/document/1_GUID- |
| | 6A0B0F3C-FA70-433C-AD55-2020BBC64718_4_en-US |
| | © 2020 USPC |
| | Page Information: |
| | USP43-NF38 - 2279 |
| | USP42-NF37 - 2229 |
| | USP41-NF36 - 2105 |
| RH | Relative Humidity: The term "relative humidity" is used herein to mean the ratio of the actual water vapor pressure at a given temperature to the vapor pressure that would occur if the air were saturated with water at the same temperature. There are many technologies for humidity measurement instruments known to the skilled person, all of which would give substantially the same RH value. |
| wt% | weight percent or percent by weight |

## SUMMARY OF THE INVENTION

Subject of the invention is a capsule shell CAPSSHELL comprising HPMC and $CaCl_2$; the amount of $CaCl_2$ comprised in CAPSSHELL is from 3'000 to 9'000 ppm based on the weight of HPMC comprised in CAPSSHELL;

CAPSSHELL does not contain a gelling agent; or
CAPSSHELL does not contain a combination of the gelling agent with a gelling aid, wherein said gelling agent is selected from the group consisting of: agar gum, guar gum, locust bean gum (carob), carrageenan, pectin, xanthan, gellan gum, konjac mannan and gelatin; and wherein said gelling aid is selected from the group consisting of: a cation such as $K^+$, $Na^+$, $Li^+$, $NH4^+$, $Ca^{2+}$ and $Mg^{2+}$.

## BRIEF DESCRIPTION OF THE DRAWINGS

The present invention will be described again with reference to the enclosed drawings, wherein:

**Figure 1:** shows a SEM picture of residual powder on the inner surface of a capsule after emptying powder from the capsule by action of DUSA equipment; so the film building polymer of the capsule shell was HPMC and the capsule shell contained $CaCl_2$

**Figure 2:** shows a SEM picture of residual powder on the inner surface of a capsule after emptying powder from the capsule by action of DUSA equipment; the film building polymer of the capsule shell was HPMC and the capsule shell did not contain $CaCl_2$

**Figure 3:**   shows a graphical representation of PR values versus LOD from the data of Table 3.

## DETAILED DESCRIPTION OF THE INVENTION

CAPSSHELL is a capsule shell for filling with a powder comprising a substance selected from the group consisting of an active pharmaceutical ingredient, medicament, and a mixture thereof. CAPSSHELL is a capsule of the type used in pharmaceutical or healthcare applications.

The HPMC and the $CaCl_2$ are contained in the capsule shell itself, that is in the wall that actually builds the capsule shell. Another word for "build" may be "constitute" or "form". The HPMC is the film forming substance that actually builds the wall, that is that builds the capsule shell itself; the $CaCl_2$ is contained in the HPMC, that is the $CaCl_2$ is contained in the wall.

The HPMC and the $CaCl_2$ meant by the invention are not meant to be contained in the content of the capsule shell, that is in the powder which is filled into the capsule shell, such as a medicament in powder form etc.

Suitable HPMC are commercially available.

The HPMC may have a methoxy content of 27.0 to 30.0 % (w/w).

The HPMC may have a hydroxypropoxy content of 4.0 to 12.0 % (w/w).

Preferably, the HPMC may have a methoxy content of 27.0- to 30.0 % (w/w) and a hydroxypropoxy content of 4.0 to 12.0 % (w/w).

In the instant invention the HPMC methoxy and hydroxypropoxy contents are expressed according to US Pharmacopeia.

There are different types of HPMC. The HPMC may for example be selected from the group consisting of

HPMC 2910 containing about 7.0 to 12.0% hydroxypropoxy group and about 28.0 to 30.0% methoxy group,
HPMC 2906 containing about 4.0 to 7.5% hydroxypropoxy group and about 27.0 to 30.0% methoxy group,
HPMC 2208 containing about 4.0 to 12.0% of hydroxypropoxy group and about 19.0 to 24.0% of methoxy group, and
HPMC 1828 containing about 23.0 to 32.0% hydroxypropoxy group and about 16.5 to 20.0% methoxy group.
The HPMC in CAPSSHELL may be one type of HPMC, it may also be a mixture of different types of HPMC.

In one embodiment the HPMC may be HPMC 2906, HPMC 2910 or a mixture thereof.

CAPSSHELL may contain from 3'500 to 9'000 ppm, preferably from 4'000 to 9'000 ppm, more preferably from 4'500 to 9'000 ppm, even more preferably from 4'750 to 9'000 ppm, especially from 5'000 to 9'000 ppm, more especially from 6'000 to 9'000 ppm, more especially from 6'000 to 8'000 ppm, in particular from 6'000 to 7'000 ppm, more in particular from 6'000 to 6'500 ppm, of $CaCl_2$, with the ppm being based on the weight of HPMC comprised in CAPSSHELL.

CAPSSHELL may contain from 3'500 to 7'000 ppm, preferably from 4'000 to 7'000 ppm, more preferably from 4'500 to 7'000 ppm, even more preferably from 4'750 to 7'000 ppm, especially from 5'000 to 7'000 ppm, more especially from 6'000 to 7'000 ppm, even more especially from 6'000 to 6'500 ppm, of $CaCl_2$, with the ppm being based on the weight of HPMC comprised in CAPSSHELL.

In another embodiment, CAPSSHELL may contain from 3'500 to 6'500 ppm, preferably from 4'000 to 6'500 ppm, more preferably from 4'500 to 6'500 ppm, even more preferably from 4'750 to 6'500 ppm, especially from 5'000 to 6'500 ppm, more especially from 6'000 to 6'500 ppm, of $CaCl_2$, with the ppm being based on the weight of HPMC comprised in CAPSSHELL.

CAPSSHELL may contain 70 wt% or more, preferably 80 wt% or more, more preferably 90 wt% or more, even more preferably 95 wt% or more, especially 97.5 wt% or more, more especially 99 wt% or more, even more especially 99.3 wt% or more, in particular 99.35 wt% or more, of HPMC, with the wt% being based on the weight of dry CAPSSHELL.

The upper limit of HPMC is 99.65 wt%, with the wt% being based on the weight of dry CAPSSHELL.

In one embodiment, dry CAPSSHELL consists of HPMC and $CaCl_2$.

In one embodiment, CAPSSHELL consists of HPMC, $CaCl_2$ and residual water, preferably with the possible amounts of $CaCl_2$ based on the weight of HPMC contained in CASPSHELL as disclosed herein, also with all their embodiments.

In one embodiment, dry CAPSSHELL consists of 99.4 to 99.35 wt% of HPMC and 0.6 to 0.66 wt% of CaClz, with the amounts of HPMC and $CaCl_2$ adding up to 100 wt%, with the wt% being based on the weight of dry CAPSSHELL.

In one embodiment, CAPSSHELL is a hard capsule shell.

The wall thickness of CAPSSHELL is known to the skilled person, a typical value may be around 100 micrometer, a typical range may be from 60 to 150 micrometer.

Typical sizes of CAPSSHELL are known to the skilled person and may be for examples expressed in the sizes 00, 0, 1, 2 or 3.

CAPSSHELL contains residual water. The residual water may stem from the production process which is using an aqueous mixture for preparing CAPSSHELL, so the water in CAPSSHELL is typically residual water remaining in CAPSSHELL after drying, and it may also stem from the humidity of the atmosphere, essentially of the air, surrounding CAPSSHELL. Typical content of residual water in CAPSSHELL is 14 wt% or less, preferably 10 wt% or less more preferably 9 wt% or less, even more preferably 7 wt% or less; the wt% being based on the weight of CAPSSHELL.

Typical ranges for the residual water content in CAPSSHELL may be from 0 to 14 wt%, preferably from 1 to 14 wt%, even more preferably from 2 to 14 wt%, especially from 2 to 10 wt%, more especially from 2 to 9 wt%, even more especially from 2 to 7 wt%, of water, the wt% being based on the weight of CAPSSHELL. CAPSSHELL does not contain a gelling agent, GELAGE, or a combination of a gelling agent, GELAGE, with a gelling aid, GELAID.

Gelling agents, GELAGE, and combinations of a gelling agent, GELAGE, with a gelling aid, GELAID, used in the manufacture of capsules are known to skilled person.

Typical gelling agents, GELAGE, are known to the skilled person, such as agar gum, guar gum, locust bean gum (carob), carrageenan, pectin, xanthan, gellan gum, konjac mannan or gelatin.

A typical gelling aid, GELAID, which may be used in combination with a gelling agent, GELAGE, is known to the skilled person and may be a cation such as $K^+$, $Na^+$, $Li^+$, $NH4^+$, $Ca^{2+}$ or $Mg^{2+}$.

CAPSSHELL may, additionally to the HPMC and the $CaCl_2$, comprise one or more additives ADD, ADD may be a viscosity modifier, defoaming aid, plasticizer, lubricant, colorant, solvent, solvent aid, surfactant, dispersant, solubilizer, stabilizer, corrective, sweetener, absorbent, adsorbent, adherent's, antioxidant, antiseptic, preservative, desiccant, flavor, perfume, pH adjuster, binder, humectant, disintegrating agent, release-controlling agent, acid, salt, or a mixture therefore.

Typical ADD may be viscosity modifier, defoaming aid, plasticizer, colorant, surfactant, dispersant, antioxidant, pH adjuster, humectant, acid, salt, or a mixture therefore.

Preferred ADD may be viscosity modifier, defoaming aid, plasticizer, colorant, surfactant, dispersant, antioxidant, pH adjuster, humectant, salt, or a mixture therefore.

Plasticizer may be glycerol, propylene glycol, sorbitol or lecithin. To avoid an excessive softness, the plasticizer content should be not too high, such as up to 2 wt%, preferably up to 1 wt%, the wt% being based on the weight of dry CAPSSHELL.

Colorant may be a pigment, such as $TiO_2$, or a dye.

Typical amount of pigment or dye may be from 0.01 to 10 wt%, preferably from 0.01 to 5 wt%, more preferably from 0.01 to 2.5 wt%, even more preferably from 0.01 to 1 wt%, the wt% being based on the weight of dry CAPSSHELL.

Dispersant may be sodium lauryl sulfate, sorbitan, or lecithin.

Antioxidant may be ascorbic acid.

Acid may be acetic acid.

Typical amount of acid may be from 0.025 to 0.75 wt%, preferably from 0.04 to 0.6 wt%, the wt% being based on the weight of HPMC.

Possible content of any ADD in CAPSSHELL may be from 0.025 to 29.65 wt%, preferably from 0.04 to 22 wt%, the wt% being based on the total weight of dry CAPSSHELL.

In one embodiment, dry CAPSSHELL consists of HPMC, $CaCl_2$. and colorant.

In one embodiment, dry CAPSSHELL consists of HPMC, $CaCl_2$. and pigment.

In one embodiment, CAPSSHELL consists of HPMC, $CaCl_2$, residual water and optionally a pigment, preferably with the possible amounts of $CaCl_2$ based on the weight of HPMC contained in CASPSHELL as disclosed herein, also with all their embodiments;

in case that a pigment is contained in CAPSSHELL then preferably with the possible amounts of HPMC based on the weight of dry CASPSHELL as disclosed herein, also with all their embodiments.

Further subject of the invention is a method for preparation of CAPSSHELL, wherein CAPSSHELL is formed by a process PROCFORMCAPS for forming a capsule shell from a mixture DIPMIX, DIPMIX is water containing $CaCl_2$ and HPMC;

with CAPSSHELL as defined herein, also with all its embodiments.

Preferably, $CaCl_2$ is present in DIPMIX in form of its solution in the water.

Preferably, HPMC is present in DIPMIX in form of its solution in the water

DIPMIX is also called a melt by the skilled person.

PROCFORMCAPS may be any conventional process for forming capsule shells known to the skilled person, such as extrusion molding, injection molding, casting or dip molding, preferably dip molding.

Dip molding can also be called dip coating.

CAPSSHELL made by dip molding comprises two parts of a capsule shell, the two parts are the called the cap and the body. These two parts are often also called two halves, but they do not necessarily need to have the same size and each of them does not necessarily need to have exactly half of the size of CAPSSHELL. Cap and body are two separate parts. When joined together they form the capsule, or the capsule shell, which can be empty or filled. The words "capsule" and capsule shell" are usually used interchangeably. The term shell refers usually to the capsule shaped polymer which forms the film which again forms the wall of the shell, that again is the shell, so the capsule shaped polymer is also called the shell. The cap may be obtained from by a mold pin having the respective geometric shape complementary to the desired shape of the cap. The body may be formed by a mold pin having the respective geometric shape complementary to the desired shape of the body. By using the respective mold pin in the dip molding either the cap or the body is obtained.

CAPSSHELL may therefore comprise two parts, the cap and the body. Said cap and body are telescopically engageable to provide CAPSSHELL. Typically, the cap and the body have each two regions, a dome shaped region, which is the closed end of the cap or of the body respectively, and an essentially cylindrically shaped region, which extends from the dome shaped region and which ends with the open end of the cap or of the body respectively.

The essentially cylindrically shaped region of the cap, or at least part of it, is telescopically engageable with the essentially cylindrically shaped region of the body, or at least with part of it. It is essentially an inserting of the body into the cap or vice versa. This inserting is typically a sliding of the cap over the body or vice versa. Typically the cap slides over the body. Thereby the essentially cylindrically shaped region of the body, or at least part of this region of the body, is inside the essentially cylindrically shaped region of the cap, or at least inside part of this region of the cap. So the essentially cylindrically regions of cap and body slide over the other one, as the case may be. So typically the body is inserted into the cap, i.e. the body slides into the cap. The telescopical engagement happens co-axial with respect to the longitudinal axis of the cap and the body.

So the telescopically engaged cap and body is the capsule.

DPIMIX needs to be provided for dip molding.

The dip molding comprises the steps of:

(1) dipping a mold pin for the first half of CAPSSHELL into DIPMIX;
(2) withdrawing the mold pin from DIPMIX while allowing a film to form on the mold pin;
(3) drying said film on the mold pin providing the first half of CPASSHELL; and
(4) removing the half of CAPSSHELL from the mold pin;

with CAPSSHELL and DIPMIX as defined herein, also with all its embodiments. CAPSSHELL comprises two parts, the two parts are called the cap and the body of CAPSSHELL.

Steps (1) to (4) are done both with a pin which is shaped to provide for the cap, and with a pin which is shaped to provide the body.

Steps (1) to (4) need to be performed in the order they are presented.

After the preparation of both parts of CAPSSHELL, both parts are joined with each other to form the capsule.

The half of a capsule shell which is removed from the mold pin may have a length which is still longer than the target length of the desired half of a capsule shell, in this case the half of the capsule shell on the mold pin and after removal from the mold pin represents an unmachined part, and is cut to the desired size to provide the desired half of a capsule shell in the desired length.

The mold pin may have an elevated temperature PINTEMP for the dip molding. In one embodiment it has an elevated temperature when it is dipped into DIPMIX and while the film is dried on the mold pin after the dipping.

PINTEMP may be 1.0 °C or more, preferably 5 °C or more, more preferably 10 °C or more, above the gelling temperature of DIPMIX. An upper limit of PINTEMP may be 95 °C. PINTEMP may be chosen according to the desired capsule size.

Typical ranges for PINTEMP may be from 45 to 95 °C, preferably from 45 to 80 °C, more preferably from 45 to 70 °C, even more preferably from 50 to 70 °C, especially from 50 to 65 °C.

Therefore before step (1), the mold pin may be pre-heated to the desired PINTEMP.

The temperature DIPMIXTEMP of DIPMIX during the dipping of the mold pins into DIPMIX may be up to 1.0 °C, preferably from 10 to 1.0 °C, more preferably from 6 to 1.0 °C, even more preferably from 6 to 2 °C, below the gelling temperature of DIPMIX.

As an example for HPMC grade 2906, DIPMIXTEMP may be from 10 to 29 °C, preferably from 15 to 29 °C, more preferably from 20 to 29 °C.

Drying of the film on the mold pin may be done by air drying. Drying may be done at elevated temperature with the temperature being above the gelling temperature of DIPMIX.

So the temperature of the air that is used for drying may be above the gelling temperature of DIPMIX.

The temperature of the air for drying of the film on the mold pin may be from 45 to 90 °C, preferably from 45 to 80 °C.

In general the duration of step (3) is 5 to 60 min.

In general step (3) is done at a RH of from 20 to 90 %, preferably, from 20 to 70 %, more preferably from 20 to 60 %.

In a preferred embodiment, drying is performed as disclosed in WO 2008/050205 A1.

DIPMIX comprises HPMC and $CaCl_2$ in amounts based on the weight of dry DIPMIX which are equivalent to the amounts of HPMC and $CaCl_2$ in CAPSSHELL based on the weight of dry CAPSSHELL as defined herein.

DIPMIX may comprise from 15 to 25 wt%, preferably from 17 to 23 wt%, more preferably from 17.5 to 22.5 wt%, of HPMC, the wt% being based on the weight of DIPMIX.

The concentration of HPMC in DIPMIX may be chosen to obtain a viscosity of DIPMIX of from 1'000 to 3'000 mPa*s, preferably of from 1'200 to 2'500 mPa*s, more preferably of from 1'600 to 2'000 mPa*s, measured at a temperature of 10 to 1.0 °C below gelling temperature of DIPMIX.

The amount of $CaCl_2$ in DIPMIX based on the weight of HPMC in DIPMIX corresponds to the content of $CaCl_2$ in dry CAPSSHELL.

DIPMIX may be prepared by a mixing MIX of a mixture CCMIX, CCMIX being a mixture of $CaCl_2$ with water, with a mixture HPMCMIX, HPMCMIX being a mixture of HMPC in water.

CCMIX may comprise from 15 to 25 wt%, preferably from 17.5 to 22.5 wt%, of CaClz, the wt% being based on the weight of CCMIX.

HPMCMIX may comprise from 15 to 25 wt%, preferably from 17.5 to 22.5 wt%, of HPMC, the wt% being based on the weight of HPMCMIX.

The amounts of HPMCMIX and CCMIX and their concentrations and the amounts of HPMC and of $CaCl_2$ are calculated and chosen in such a way that the desired amounts of $CaCl_2$ and of HPMC in DIPMIX is provided in order to provide for the desired amounts of $CaCl_2$ and of HPMC in CAPSSHELL.

The amounts of $CaCl_2$ and of HPMC in dry DIPMIX are equivalent to the respective amounts in dry CAPSSHELL.

CCMIX may be prepared by a mixing MIXCC of $CaCl_2$ with water.

HPMCMIX may be prepared by a mixing MIXHPMC of HPMC with water.

The water may be at a temperature above room temperature, preferably above 60 °C, more preferably above 70 °C. Optimal temperatures can be determined by the skilled person. The mixing of HPMC with water at a temperature above 60 °C provides a dispersion of HPMC in water. The dispersion may be cooled to a temperature of from 10 to 20 °C to achieve the dissolution of the HPMC.

The gelling temperature of any solution of HPMC in water, such as HPMCMIX or DIPMIX, may be determined by a measurement of the viscosity by progressively heating the solution. The temperature at which the viscosity starts to sharply increase is considered as the gelling temperature. As an example, for a concentration of about 19 wt% in water, any HPMC of the invention fulfilling the USP definition of HPMC type 2906 has a gelling temperature of about between 30 and 40 °C. As an additional example, for concentrations between 15 and 25 wt% in water, an HPMC of the invention fulfilling the USP definition of HPMC with a hydroxypropoxy content of about 6%, has a gelling temperature between about 30 and 40°C.

Further subject of the invention is CAPSSHELL filled with a formulation FILLFORM comprising an active ingredient ACTINGR, ACTINGR may be selected from the group consisting of active pharmaceutical ingredient, medicament, and a mixture thereof;
with CAPSSHELL as defined herein, also with all its embodiments.

FILLFORM may have the form of a powder.

Further subject of the invention is the use of CAPSSHELL for filling with FILLFORM, with CAPSSHELL and FILLFORM as defined herein, also with all its embodiments.

FILLFORM may comprise ACTINGR in an amount from 0.05 to 100 wt%, preferably from 0.5 to 90 wt%, more preferably from 1 to 50 wt%, even more preferably from 5 to 30 wt%, the wt% being based on the weight of dry FILLFORM.

Examples for medicaments or for API which are candidates for ACTINGR to be filled in CASPSSHELL are those which are typically used in DPI applications and are known to the skilled person, such as from the class of mucolytics, bronchodilators, corticosteroids, xanthine derivatives, leukotriene antagonists, proteins or peptides, and mixtures thereof.

Further subject of the invention is the use of CAPSSHELL in dry powder inhalers, with CAPSSHELL as defined herein, also with all its embodiments.

For this use of CAPSSHELL in dry powder inhalers, CAPSSHELL in form of a capsule filled with FILLFORM is used in dry powder inhalers for dispensing the FILLFORM during the action of the dry powder inhaler. FILLFORM is released from CAPSSHELL by action of the dry powder inhaler upon CAPSSHELL. The release of FILLFORM provides for inhalation of FILLFORM by the patient

Further subject of the invention is a dry powder inhaler with CAPSSHELL inserted in the dry powder inhaler, preferably with CAPSSHELL filled with FILLFORM, with CAPSSHELL and FILLFORM as defined herein, also with all their embodiments.

**EXAMPLES**

**Materials, Apparatus, Methods and further Abbreviations used in this specification**

| | |
|---|---|
| CC | Calcium Chloride as Calcium chloride dihydrate, $CaCl_2.2H_2O$, CAS 10035-04-8, Product 22317.297, titre 99.8%, from VWR International bvba, 3001 Leuven, Belgium |
| HPMC | Hydroxypropyl methylcellulose, also called Hypromellose, grade 2906, METHOCEU‴ FS Premium LV Hydroxypropyl Methylcellulose, 'T'he Dow Chemical Company, SWITZERLAND, 29.5 % Methoxyl, 6.2 % Hydroxypropoxyl |
| lactose blend | lactose for inhalation in a quality which is obtainable as Respitose ML001 from DFE pharma, 47568 Goch, Germany: d10 or 4 micrometer, D50 of 49 micrometer and d90 of 169 micrometer, data of particle size distribution obtained from Murphy, Seamus. (2014). UNDERSTANDING THE AFFECT OF DPI DEVICE AND LACTOSE TYPE ON THE OUTPUT FROM A DEVICE. Journal of Aerosol Medicine and Pulmonary Drug Delivery. 27. A16-A16. |
| LOD | loss on drying |

APPARATUS

➢ 1 balance Mettler Toledo type AB204
➢ 1 drying oven type Memmert U40
➢ 1 aluminium container

PROCEDURE

To measure the LOD proceed as follows:

➢ Weight 1 +/-0.001 g of capsules material in a pre-weighed aluminum container
➢ Place the container in the drying oven set up to 100 to 105 °C and cover the container with a wire gauze
➢ Store and dry for 18 h at 100 to 105 °C
➢ Cool in a dessiccator equipped with dessiccant and then after max. 30 min weigh the capsules
➢ Calculate the loss on drying as a percentage of original weight
➢ Reproduce 3 times, if needed, and evaluate the accuracy of the determination of the LOD.

| | |
|---|---|
| PR | powder retention |
| SD | standard deviation |
| SML | Sorbitan monolaurate, Glycomul L KFG(Non-GMO)/SCHL-470LB, Acid value 6, Color-Gardner 1963 is 16, Hydroxyl value 358, Saponification value 165, Water Content by KF 1.3, Lonza, 3930 Visp, Switzerland |
| RF10 | SOLEC(TM) RF-10 Standard Rapeseed Lecithin Fluid, Acid Value 28.60, Acetone Insolubles 62.80 %, Solae Europe, S.A., 2, 1218 Le Grand Saconnex, Switzerland |
| Tube tester | Capsugel, developed in-house: |

**Method:**

The capsule's fracture and elasticity behavior is measured by its resistance to an impact test with a tube.

**PROCEDURE**

1. Store the capsules for five days before testing in desiccators.

   Storage conditions available are: 10, 23, 33, 45 % RH

Store 50 capsules for each condition in opened boxes. Closes it thereafter to avoid any moisture exchange with the surrounding atmosphere.

2. Place a capsule horizontally on a flat surface.
3. Put the weight of 100 g in the tube.
4. Place the tube on the capsule and push the latch to release the weight.
5. Test 50 capsules.

Use closed boxes for storing after equilibration with chosen RH, and do not take out every 50 capsules at the same time (to avoid any humidity retaking).

Record the number of body, cap or capsule (body and cap) broken.

The tube tester and the test method are disclosed in M. Sherry Ku et al., "Performance qualification of a new hypromellose capsule: Part I. Comparative evaluation of physical, mechanical and processability quality attributes of Vcaps Plus®, Quali-V® and gelatin capsules", International Journal of Pharmaceutics, Volume 386, Issues 1-2, 15 February 2010, Pages 30-41, chapter 2.5. Mechanical strength evaluation

| | |
|---|---|
| Balance | XPE205DR, Mettler-Toledo AG |
| Friabilator | Friability/Abrasion Tester TAR of ERWEKA GmbH, 63225 Langen, Germany |
| DUSA | Dosage Unit Sampling Apparatus, an equipment to test inhalation products, equipped with critical flow controller which comes with the product model name TPK 2000, HCP5 vacuum pumps, filter PALLFLEX 47 mm and DFM 2000 flow meter, Copley Scientific, also called an apparatus for DDU (Delivered Dose Uniformity) for Dry Powder Inhalers by Copley Scientific |
| SEM | Scanning Electron Microscope FlexSEM 1000 |

## Example 1: Preparation of CC melt

### Step 1: Preparation of HPMC melt (HPMCMIX)

List of compounds to prepare HPMC melt:

| | |
|---|---|
| HPMC: | 20.55 wt% |
| Water | 79.45 wt% |

Preparation of HPMC melt was done by filling the vessel with water of 80 to 85 °C into which HPMC is charged and mixed. Then the vessel was cooled to 10 to 20 °C solubilize the HPMC. After one hour at 10 to 20 °C the HPMC melt is heated to and maintained at 29 °C for further use.

### Step 2: Preparation of solution of $CaCl_2.2H_2O$ (CCMIX)

$CaCl_2.2H_2O$ was added into the water of 80 °C under stirring of 200 rpm.

### Step 3: Preparation of CC melt (DIPMIX)

The CC melt is a mixture of the HPMC melt, prepared according to Step 1, and the solution of $CaCl_2.2H_2O$, prepared according to Step 2.

The solution of $CaCl_2.2H_2O$ was mixed with the HPMC melt prepared according to Step 1, thereby providing a CC melt.

Three CC melts, CC-A* melt, CC-A** and CC-B melt, were prepared in this way, a fourth melt, CC-A melt, is prepared in this way, the amounts for these CC melts are given in Table 1.

| | Table 1 | | | | |
|---|---|---|---|---|---|
| Step 1 | Preparation of HPMC melt | CC-A | CC-A* | CC-A** | CC-B |
| | HPMC | 57.54 kg | 59.82 kg | 49.53 kg | 1027.5 g |
| | water | 222.43 kg | 231.23 kg | 191.47 kg | 3972.24 g |

(continued)

|  | | Table 1 | CC-A | CC-A* | CC-A** | CC-B |
|---|---|---|---|---|---|---|
| Step 1 | | **Preparation of HPMC melt** | CC-A | CC-A* | CC-A** | CC-B |
| | | Total weight | 279.97 kg | 291.07 kg | 241 kg | 4999.74 g |
| | | content of HPMC | 20.55 wt% | 20.55 wt% | 20.55 wt% | 20.55 wt% |
| | | | | | | |
| Step 2 | | **Preparation of solution of $CaCl_2.2H_2O$** | | | | |
| | | $CaCl_2.2H_2O$ | 503.41 g | 503.41 g | 497.05 g | 12.07 g |
| | | water | 2013.68 g | 2013.68 g | 1988.2 g | 48.28 g |
| | | Total weight | 2517.09 g | 2517.09 g | 2485.25 g | 60.35 g |
| | | content of $CaCl_2.2H_2O$ | 20 wt% | 20 wt% | 20 wt% | 20 wt% |
| | | | | | | |
| Step 3 | | **Preparation of CC melt** | | | | |
| | | HPMC melt, prepared in line with the procedure given in Step 1 | 280 kg | 291.07 kg | 241 kg | 4500 g |
| | | Solution of $CaCl_2.2H_2O$, prepared according to Step 2 | 2517.09 g | 2517.09 g | 2485.25 g | 59.59 g |
| | | $CaCl_2$ content | 6'561 ppm based on weight of HPMC + $CaCl_2$ | 6'313 ppm based on dry weight of HPMC + $CaCl_2$ | 7'519 ppm based on weight of HPMC + $CaCl_2$ | 9'639 ppm based on weight of HPMC + $CaCl_2$ |
| | | | 6'605 ppm based on weight of HPMC | 6'353 ppm based on weight of HPMC | 7'576 ppm based on weight of HPMC | 9'732 ppm based on weight of HPMC |

(continued)

| Step 3 | Preparation of CC melt | | | | |
|---|---|---|---|---|---|
| | HPMC content | 20.55 wt% based on weight of HPMC melt | 20.55 wt% based on weight of HPMC melt | 20.55 wt% based on weight of HPMC melt | 20.55 wt% based on weight of HPMC melt |

MW of $CaCl_2$ = 110.98 g/mol
MW of $CaCl_2.2H_2O$ = 147.01 g/mol
Weight of $CaCl_2$:
CC-A: 503.41 g * (110.98 / 147.01) = 380.03 g
CC-A*: 503.41 g * (110.98 / 147.01) = 380.03 g
CC-A**: 497.05 g * (110.98 / 147.01) = 375.23 g
CC-B: 59.59 g 20 wt% = 11.918 g $CaCl_2.2H_2O$
11.918 g * (110.98 / 147.01) = 9.0 g
ppm of $CaCl_2$ based on weight of HPMC + $CaCl_2$:
CC-A: 380.03 g / (57'540 g + 380.03 g) * 1'000'000 = 6'561 ppm
CC-A*: 380.03 g / (59'820 g + 380.03 g) * 1'000'000 = 6'313 ppm
CC-A**: 375.23 g / (49'530 g + 375.23 g) * 1'000'000 = 7'519 ppm
CC-B: 4'500 g 20.55 wt% = 924.75 g HPMC
9.0 g / (924.75 g + 9.0 g) * 1'000'000 = 9'639 ppm
ppm of $CaCl_2$ based on weight of HPMC:
CC-A: 380.03 g / 57'540 g * 1'000'000 = 6'605 ppm
CC-A*: 380.03 g / 59'820 g * 1'000'000 = 6'353 ppm
CC-A**: 375.23 g / 49'530 g * 1'000'000 = 7'576 ppm
CC-B: 9.0 g / 924.75 g * 1'000'000 = 9'732 ppm (not according to the invention)

## Example 2: Preparation of CC shell and CC capsules and of HPMC shell and HPMC capsules

The respective CC melts CC-A* and CC-B, prepared according to example 1, and the HPMC melt, prepared according to examples 1 step 1, were used to produce CC shells and HPMC shells in form of capsule halves (bodies and caps) with capsule size 3 and standard target weights (capsule weight 47 +/- 3 mg) through conventional mold dipping by dipping stainless steel mold pins with a temperature of 55 °C into the respective CC melt or HPMC melt respectively with a temperature of 29 °C. A film was formed on the mold pins. After first drying of the film on the mold pins at 50 to 60 °C and 30 to 40 % RH for 15 to 20 min, and a second drying at 50 to 60 °C and 25 to 30 % RH for 30 min, CC capsule halves and HPMC capsule halves respectively were obtained.
Capsules were assembled by joining always one cap with one body.

## Example 3: Testing of capsule - mechanical performance

The fracture behavior of the capsules prepared according to Example 2, was measured by its resistance to an impact test with a tube tester. In preparation of the test the capsules were stored for equilibration for five days under four storage conditions in desiccators: 10, 23, 33 and 45 % RH in order to obtain capsules with different LOD. Number of capsules tested was 50 per each RH value and the number of broken body or broken cap was recorded. Percentage of broken capsules is presented in Table 2.

| Table 2 | | |
|---|---|---|
| CC melt | [%] broken capsules (1) | Storage condition RH [%[ |
| CC-A* (6'353 ppm $CaCl_2$) | 7±6 | 10 |
| | 1±2 | 23 |
| | 0 | 33 |
| | 0 | 45 |

(continued)

| Table 2 | | |
|---|---|---|
| **CC melt** | **[%] broken capsules (1)** | **Storage condition RH [%[** |
| CC-A** (7'576 ppm CaCl$_2$) | 9±4 | 10 |
| | 0 | 23 |
| | 0 | 33 |
| | 0 | 45 |
| CC-B (9'732 ppm CaCl$_2$) | 66±6 | 10 |
| | 7±3 | 23 |
| | 1±1 | 33 |
| | 0 | 45 |
| HPMC capsule | 4±2 | 10 |
| | 1±1 | 23 |
| | 0 | 33 |
| | 0 | 45 |

(1) Values in table 2 represent mean ± standard deviation of 3 seperate tests.

When the amount of CaCl$_2$ is too high the mechanical performance deteriorates.

**Example 4: Testing on capsules - powder retention PR in capsules**

To determine residual powder in capsules DUSA equipment from Copley Scientific was used. Capsules were equilibrated to different LOD by storage in desiccators at 23, 33, 45 and 50% RH. Ten capsules, prepared according to Example 2, were filled with 25 +/- 1 mg of lactose blend. These ten capsules filled with lactose blend were placed in a plastic bottle with a volume of 25 ml with a lid and tumbled in a friabilator TAR for 100 times. DUSA device was used to empty the capsules. Its set up was adjusted according to USP Pharmacopea guidelines section 601, heading "Sampling the Delivered Dose from Dry Powered Inhalers" Apparatus B; two pumps were used with the flow parameters of 100 L/min and time of aspiration of 2.4 s in order to match the volume of air of 4 L as required by the cited USP Pharmacopeia guideline section 601, to be withdrawn from the inhaler. Of this total often capsules the weight before filling with powder (Wempty) and after emptying (Wemptied) by the DUSA device was determined. The total amount of powder that was filled into these ten capsules was also recorded (Wpowder). PR is the percentage of residual powder and was determined from the equation EQ1:

$$EQ1: \qquad \% \ PR = 100 * [ \ (Wemptied - Wempty) / Wpowder \ ]$$

Results represent mean and standard deviation SD of 3 samples, each of a total 10 capsules tested and are given in the Table 3:

| Table 3 | | | | | |
|---|---|---|---|---|---|
| **LOD [%] and**<br><br>**Storage condition RH [%]** | **HPMC capsule**<br><br>**PR [%] mean +/- SD** | **CC-A* capsule (6'353 ppm CaCl$_2$)**<br><br>**PR [%] mean +/- SD** | **CC-A** capsule (7'576 ppm CaCl$_2$)**<br><br>**PR [%] mean +/- SD** | **CC-B capsule (9'732 ppm CaCl$_2$)**<br><br>**PR [%] mean +/- SD** | **US 5,626,871 Example 17**<br><br>**PR [%] mean +/- SD** |
| **2.5 to 3.0 23** | 1.95+/-0.1 | 1.89+/-0.2 | 1.8+/-0.2 | 1.5+/-0.5 | 1.6+/-0.2 |

(continued)

| Table 3 | | | | | |
|---|---|---|---|---|---|
| LOD [%] and<br><br>Storage condition RH [%] | HPMC capsule<br><br>PR [%] mean +/- SD | CC-A* capsule (6'353 ppm CaCl$_2$)<br><br>PR [%] mean +/- SD | CC-A** capsule (7'576 ppm CaCl$_2$)<br><br>PR [%] mean +/- SD | CC-B capsule (9'732 ppm CaCl$_2$)<br><br>PR [%] mean +/- SD | US 5,626,871 Example 17<br><br>PR [%] mean +/- SD |
| 3.5 to 4.0<br>33 | 1.27+/-0.1 | 1.12+/-0.1 | 1.2+/-0.1 | 1.2+/-0.1 | 1.2+/-0.2 |
| 4.0 to 4.5<br>45 | 1.33+/-0.1 | 0.8+/-0.1 | 0.6+/-0.04 | 0.4+/-0.2 | 0.7+/-0.3 |
| 4.5 to 5.0<br>50 | 0.62+/-0.1 | 0.19+/-0.1 | 0.5+/-0.2 | 0.8+/-0.2 | 0.6+/-0.03 |
| 5.0 to 5.5 (*) | 0.41+/-0.3 | below 0.04 | 0.2+/-0.03 | 0.2+/-0.1 | 0.2+/-0.04 |
| (*) The capsule samples with LOD from 5.0 to 5.5. were obtained without prior equilibration by any storage in desiccators at defined RH; rather by letting them stand at the RH present in the laboratory of ca. 50 % RH and temperature of ca. 22 °C and the given LOD was the one attained by this treatment. | | | | | |

Figure 3 shows a graphical representation of PR values versus LOD from the data of Table 3. Individual values are shown in black and mean values in grey.

▲ Triangle:        Capsules made from CC-A* melt are represented by a triangle.
● Bullet:        Capsules made from CC-A** melt are represented by a bullet.
■ Square:        Capsules made from CC-B melt are represented by a square.
♦ Rhombus:        Capsules which were used in US 5,626,871 Example 17 (medical hard capsules composed essentially of hydroxypropyl methyl cellulose [composition: 93 parts by weight of hydroxypropyl methyl cellulose, "TC-5R" produced by Shinetsu Kagaku; 1 part by weight of carrageenan; 1 part by weight of potassium chloride; 5 parts by weight of water]) are represented by a square.
○ Circle:        HPMC capsules are represented by a triangle.

Results:

- HPMC capsules show the highest mean PR values compared to capsules made from CC-A* melt and from CC-A** melt.
- Capsules made from CC-A* melt and from CC-A** melt show either comparable or better PR values compared to the capsules with a gelling agent used in US 5,626,871 Example 17.
- The capsules made from CC-A* melt and from CC-A** melt show consistently lower mean PR values than capsules made from HPMC melt.

**Example 5: Testing on capsules - SEM imaging of capsules internal surface after DUSA test**

CC-A* capsules and HPMC capsules with LOD from 4.5 to 5.0, prepared according to Example 2 and storage in desiccator at 50% RH as described in Example 4, were also tested using powder that was obtained by purchasing a publicly available medicament in form of a capsule filled with powder in a pharmacy and extracting this powder and filling it into the capsules. Then the capsules were emptied by action of DUSA equipment as described in Example 4 and any residual powder was determined by visual inspection. Visual inspection showed that CC-A* capsules contained significantly less powder compared to HPMC capsules.

The PR was 0.6 % for the HPMC capsules and 0.11 % for the CC-A* capsules.

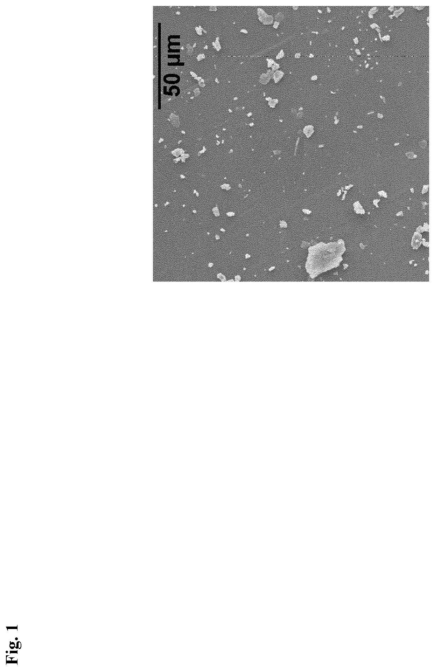

Figure 1 shows a SEM picture of residual powder on the inner surface of one such CC-A* capsule after emptying powder from the capsule by action of DUSA equipment; so the film building polymer of the capsule shell was HPMC and the capsule shell contained $CaCl_2$.

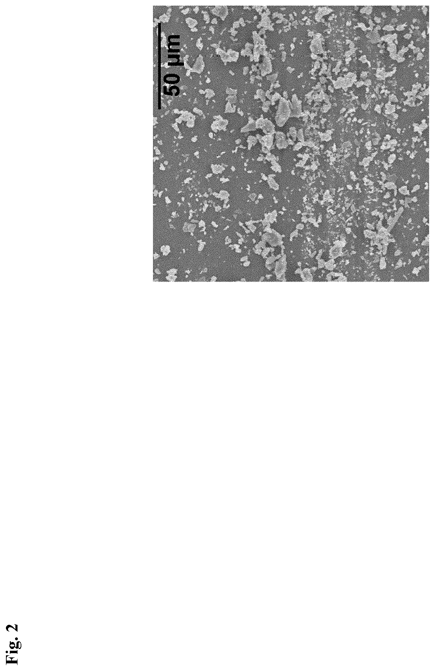

Figure 2: shows a SEM picture of residual powder on the inner surface of a capsule after emptying powder from the capsule by action of DUSA equipment; so the film building polymer of the capsule shell was HPMC and the capsule shell did not contain $CaCl_2$.

Obviously Fig. 1 shows much lower number of powder particles than Fig 2.

### Example 6: Testing of capsules - Puncture Test

A puncture test was done with the capsules made from CC-A* melt and the capsules which were used in US 5,626,871 Example 17 (medical hard capsules composed essentially of hydroxypropyl methyl cellulose [composition: 93 parts by weight of hydroxypropyl methyl cellulose, "TC-5R" produced by Shinetsu Kagaku; 1 part by weight of carrageenan; 1 part by weight of potassium chloride; 5 parts by weight of water]).

Protocol of the puncture test:

- Capsules (N = 15) were equilibrated in desiccators at 23 and 45 % RH storage conditions during 5 days.
- For the puncture test Plastiape's RS01 Dry Powder Inhaler (Plastiape S.p.a. con socio unico, Osnago, Italy) was used.
- A capsule was placed inside the inhaler chamber and punctured by pushing onto it the two lateral needles of the inhaler at the same time.

Once punctured, capsules are one by one checked visually and capsules not qualifying were identified which were capsules whose operculum was not pierced or operculum of the capsule or some of its parts are detached risking to deposit some capsules particles together with powder in the patient by the inhalation.

Sorted capsules are counted and presented as a percentage of total capsules analysed, results are given in Table 6.

| Table 6 | | |
|---|---|---|
| Sample | Not qualifying (23 % RH [%]) | Not qualifying (45 % RH [%]) |
| US 5,626,871 **Example 17** | 7 | 7 |
| **CC-A*** | 0 | 0 |
| **CC-A**** | 0 | 0 |

Capsules made from CC-A* melt and from CC-A** melt show significantly better performance in the puncture test compared to the capsules with a gelling agent used in US 5,626,871 Example 17, no not qualifying capsules were observed.

### Comparative Examples with Additives

### Preparation of additive melts and of capsules thereof

Two other additives were tested instead to $CaCl_2.2H_2O$.

The additives were:

- Sorbitan monolaurate SML

- Rapeseed lecithin RF 10

The content of the additive in the respective melt was 5000 ppm on weight of HPMC and the respective melts were prepared according to the procedure of example 1 with the difference in step 2 and with the amounts given in Table 4, and in step 3 the respective dispersion of additive was used. instead of the solution of $CaCl_2.2H_2O$

### Step 2 Preparation of dispersion of additive

The additive was added into the water and subjected to homogenization by an Ultra-Turrax, IKA T25 with 8000 to 9000 rpm speed for 5 min.

The SML dispersion was left to stand until the foam had vanished prior to the addition into HPMC melt, while the dispersion of RF 10 was used directly without the need of letting it stand since no foam had formed.

| | | Table 4 | |
|---|---|---|---|
| | Step 2 | **Preparation of dispersion of additive** | |
| | | additive | 10 g |
| | | water | 90 g |
| | | content of additive | 10 wt% |
| | Step 3 | **Preparation of additive melt** | |
| | | HPMC melt prepared accordin got Example 1, step 1 | 4404.8 g |
| | | Dispersion of additive prepared accordin to step 2 | 45.15 g |
| | | additive content | 5000 ppm based on weight of HPMC |
| | | HPMC content | 20.5 wt% based on weight of HPMC melt |

The preparation of additive melts provided two melts: RF10 melt and SML melt. With these two additive melts then additive capsules shells were prepared according to the method of dip molding as described in example 2.

**Testing on additive capsules prepared from additives melts - powder retention PR in capsules**

PR by the additive capsules with an LOD (%) of 5.0 to 5.5 was tested according to the method described in Example 4. The LOD of 5.0 to 5.5 % was obtained as described under remark (*). Table 5 shows the PR values.

| Table 5 | |
|---|---|
| **Shell** | **PR [%] mean +/- SD** |
| **HPMC capsule, see example 4** | 0.41+/-0.3 |

| | see example 4 |
|---|---|
| **SML capsule** | 0.35 ± 0.1 |
| **RF10 capsule** | 0.25 ± 0.1 |
| **CC-A\* capsule, see example 4** | below 0.04 see example 4 |

**Claims**

1.  A capsule shell CAPSSHELL comprising HPMC and $CaCl_2$;

the amount of $CaCl_2$ comprised in CAPSSHELL is from 3'000 to 9'000 ppm based on the weight of HPMC comprised in CAPSSHELL;
CAPSSHELL does not contain a gelling agent; or
CAPSSHELL does not contain a combination of the gelling agent with a gelling aid,
wherein said gelling agent is selected from the group consisting of: agar gum, guar gum, locust bean gum (carob), carrageenan, pectin, xanthan, gellan gum, konjac mannan and gelatin; and
wherein said gelling aid is selected from the group consisting of: a cation such as $K^+$, $Na^+$, $Li^+$, $NH4^+$, $Ca^{2+}$ and $Mg^{2+}$.

2.  CAPSSHELL according to claim 1, wherein

the HPMC is selected from the group consisting of

HPMC 2910 containing about 7.0 to 12.0% hydroxypropoxy group and about 28.0 to 30.0% methoxy group,
HPMC 2906 containing about 4.0 to 7.5% hydroxypropoxy group and about 27.0 to 30.0% methoxy group,
HPMC 2208 containing about 4.0 to 12.0% of hydroxypropoxy group and about 19.0 to 24.0% of methoxy group, and
HPMC 1828 containing about 23.0 to 32.0% hydroxypropoxy group and about 16.5 to 20.0% methoxy group.

**3.** CAPSSHELL according to claim 1, wherein
the HPMC has a methoxy content of 27.0 to 30.0 % (w/w).

**4.** CAPSSHELL according to claim 1 or 2, wherein
the HPMC has a hydroxypropoxy content of 4.0 to 12.0 % (w/w).

**5.** CAPSSHELL according to one or more of claims 1 to 4, wherein
the HPMC is HPMC 2906, HPMC 2910 or a mixture thereof.

**6.** CAPS SHELL according to one or more of claims 1 to 5, wherein
CAPSSHELL contains from 3'500 to 9'000 ppm of $CaCl_2$, with the ppm being based on the weight of HPMC comprised in CAPSSHELL.

**7.** CAPSSHELL according to one or more of claims 1 to 6, wherein
CAPSSHELL contains from 4'000 to 9'000 ppm of $CaCl_2$, with the ppm being based on the weight of HPMC comprised in CAPSSHELL.

**8.** CAPSSHELL according to one or more of claims 1 to 7, wherein
CAPSSHELL contains 70 wt% or more of HPMC, with the wt% being based on the weight of dry CAPSSHELL.

**9.** CAPSSHELL according to one or more of claims 1 to 8, wherein
CAPSSHELL comprises one or more additives ADD, ADD may be a viscosity modifier, defoaming aid, plasticizer, lubricant, colorant, solvent, solvent aid, surfactant, dispersant, solubilizer, stabilizer, corrective, sweetener, absorbent, adsorbent, adherent's, antioxidant, antiseptic, preservative, desiccant, flavor, perfume, pH adjuster, binder, humectant, disintegrating agent, release-controlling agent, acid, salt, or a mixture therefore.

**10.** CAPSSHELL according to one or more of claims 1 to 8, wherein
dry CAPSSHELL consists of HPMC and $CaCl_2$.

**11.** CAPSSHELL according to one or more of claims 1 to 8, wherein
dry CAPSSHELL consists of 99.4 to 99.35 wt% of HPMC and 0.6 to 0.66 wt% of $CaCl_2$, with the amounts of HPMC and $CaCl_2$ adding up to 100 wt%, with the wt% being based on the weight of dry CAPSSHELL.

**12.** CAPSSHELL according to one or more of claims 1 to 9, wherein
dry CAPSSHELL consists of HPMC, $CaCl_2$. and colorant.

**13.** A method for preparation of CAPSSHELL, wherein

CAPS SHELL is formed by a process PROCFORMCAPS for forming a capsule shell from a mixture DIPMIX, DIPMIX is water containing $CaCl_2$ and HPMC;
with CAPSSHELL as defined in claim 1.

**14.** A method for preparation of CAPS SHELL according to claim 13, wherein PROCFORMCAPS is extrusion molding, injection molding, casting or dip molding.

**15.** A method for preparation of CAPS SHELL according to claim 13 or 14, wherein PROCFORMCAPS is dip molding

**16.** A method for preparation of CAPSSHELL according to claim 15, wherein

the dip molding comprises the steps of:

(1) dipping a mold pin for the first half of CAPSSHELL into DIPMIX;

(2) withdrawing the mold pin from DIPMIX while allowing a film to form on the mold pin;
(3) drying said film on the mold pin providing the first half of CPASSHELL; and
(4) removing the half of CAPSSHELL from the mold pin;

with CAPSSHELL as defined in claim 1 and DIPMIX as defined in claim 13;
and wherein
CAPSSHELL comprises two parts, the two parts are called the cap and the body of CAPS SHELL;
steps (1) to (4) are done both with a pin which is shaped to provide for the cap, and with a pin which is shaped to provide the body.

17. CAPSSHELL filled with a formulation FILLFORM comprising an active ingredient ACTINGR, ACTINGR may be selected from the group consisting of active pharmaceutical ingredient, medicament, and a mixture thereof; with CAPSSHELL as defined in one or more of claims 1 to 12.

18. The use of CAPSSHELL for filling with FILLFORM, with CAPSSHELL as defined in claim 1 and FILLFORM as defined in claim 17.

19. The use of CAPSSHELL in dry powder inhalers, with CAPSSHELL as defined in one or more of claims 1 to 12 and 17.

20. A dry powder inhaler with CAPSSHELL inserted in the dry powder inhaler, with CAPSSHELL as defined in one or more of claims 1 to 12 and 17.


**Patentansprüche**

1. Kapselhülle CAPSSHELL, umfassend HPMC und $CaCl_2$;

   die in CAPSSHELL enthaltene $CaCl_2$-Menge beträgt 3.000 bis 9.000 ppm, basierend auf dem Gewicht von HPMC, die in CAPSSHELL enthalten ist;
   CAPSSHELL enthält kein Geliermittel; oder
   CAPSSHELL enthält keine Kombination des Geliermittels mit einem Gelierhilfsmittel,
   wobei das Geliermittel aus der Gruppe ausgewählt ist, die aus Folgendem besteht: Agar-Gummi, Guar-Gummi, Johannisbrotkernmehl (Johannisbrot), Carrageen, Pektin, Xanthan, Gellan-Gummi, Konjak-Mannan und Gelatine;
   und
   wobei das Gelierhilfsmittel aus der Gruppe ausgewählt ist, die aus Folgendem besteht: einem Kation wie $K^+$, $Na^+$, $Li^+$, $NH4^+$, $Ca^{2+}$ und $Mg^{2+}$.

2. CAPSSHELL nach Anspruch 1, wobei

   die HPMC aus der Gruppe ausgewählt ist, die aus Folgendem besteht
   HPMC 2910, enthaltend etwa 7,0 bis 12,0 % Hydroxypropoxygruppe und etwa 28,0 bis 30,0 % Methoxygruppe,
   HPMC 2906, enthaltend etwa 4,0 bis 7,5 % Hydroxypropoxygruppe und etwa 27,0 bis 30,0 % Methoxygruppe,
   HPMC 2208, enthaltend etwa 4,0 bis 12,0 % Hydroxypropoxygruppe und etwa 19,0 bis 24,0 % Methoxygruppe,
   und
   HPMC 1828, enthaltend etwa 23,0 bis 32,0 % Hydroxypropoxygruppe und etwa 16,5 bis 20,0 % Methoxygruppe.

3. CAPSSHELL nach Anspruch 1, wobei
   die HPMC einen Methoxygehalt von 27,0 bis 30,0 % (Gew./Gew.) aufweist.

4. CAPSSHELL nach Anspruch 1 oder 2, wobei
   die HPMC einen Hydroxypropoxygehalt von 4,0 bis 12,0 % (Gew./Gew.) aufweist.

5. CAPSSHELL nach einem oder mehreren der Ansprüche 1 bis 4, wobei
   die HPMC HPMC 2906, HPMC 2910 oder eine Mischung davon ist.

6. CAPSSHELL nach einem oder mehreren der Ansprüche 1 bis 5, wobei
   CAPSSHELL 3.500 bis 9.000 ppm $CaCl_2$ enthält, wobei die ppm auf dem Gewicht der in CAPSSHELL enthaltenen HPMC basieren.

7. CAPSSHELL nach einem oder mehreren der Ansprüche 1 bis 6, wobei
CAPSSHELL 4.000 bis 9.000 ppm $CaCl_2$ enthält, wobei die ppm auf dem Gewicht der in CAPSSHELL enthaltenen HPMC basieren.

8. CAPSSHELL nach einem oder mehreren der Ansprüche 1 bis 7, wobei
CAPSSHELL 70 Gew.-% oder mehr HPMC enthält, wobei die Gew.-% auf dem Gewicht von trockener CAPSSHELL basieren.

9. CAPSSHELL nach einem oder mehreren der Ansprüche 1 bis 8, wobei
CAPSSHELL einen oder mehrere Zusatzstoffe ADD umfasst, wobei ADD ein Viskositätsmodifikator, Entschäumungshilfsmittel, Weichmacher, Schmiermittel, Farbstoff, Lösungsmittel, Lösungshilfsmittel, Tensid, Dispergiermittel, Lösungsvermittler, Stabilisator, Korrektiv, Süßstoff, Absorptionsmittel, Adsorptionsmittel, Haftmittel, Antioxidans, Antiseptikum, Konservierungsmittel, Trockenmittel, Aroma, Duftstoff, Mittel zur Einstellung des pH-Werts, Bindemittel, Feuchthaltemittel, Sprengmittel, Mittel zur Steuerung der Freisetzung, eine Säure, ein Salz oder eine Mischung davon sein kann.

10. CAPSSHELL nach einem oder mehreren der Ansprüche 1 bis 8, wobei
trockene CAPSSHELL aus HPMC und $CaCl_2$ besteht.

11. CAPSSHELL nach einem oder mehreren der Ansprüche 1 bis 8, wobei
trockene CAPSSHELL aus 99,4 bis 99,35 Gew.-% HPMC und 0,6 bis 0,66 Gew.-% $CaCl_2$ besteht, wobei die Menge an HPMC und $CaCl_2$ zu 100 Gew.-% aufaddieren, wobei das Gew.-% auf dem Gewicht von trockener CAPSSHELL basiert.

12. CAPSSHELL nach einem oder mehreren der Ansprüche 1 bis 9, wobei
trockene CAPSSHELL aus HPMC, $CaCl_2$ und Farbstoff besteht.

13. Verfahren zur Herstellung von CAPSSHELL, wobei

CAPSSHELL durch einen Prozess PROCFORMCAPS zum Bilden einer Kapselhülle aus einer Mischung DIPMIX gebildet wird, DIPMIX Wasser ist, das $CaCl_2$ und HPMC enthält;
wobei CAPSSHELL wie in Anspruch 1 definiert ist.

14. Verfahren zur Herstellung von CAPSSHELL nach Anspruch 13, wobei
PROCFORMCAPS Strangpressen, Spritzgießen, Gießen oder Tauchformen ist.

15. Verfahren zur Herstellung von CAPSSHELL nach Anspruch 13 oder 14,
wobei PROCFORMCAPS Tauchformen ist.

16. Verfahren zur Herstellung von CAPSSHELL nach Anspruch 15,

wobei das Tauchformen die folgenden Schritte umfasst:

(1) Eintauchen eines Formstifts für die erste Hälfte von CAPSSHELL in DIPMIX;
(2) Herausziehen des Formstifts aus DIPMIX, während sich ein Film auf dem Formstift bilden kann;
(3) Trocknen des Films auf dem Formstift, wodurch die erste Hälfte von CPASSHELL bereitgestellt wird; und
(4) Entfernen der Hälfte von CAPSSHELL von dem Formstift;

wobei CAPSSHELL wie in Anspruch 1 definiert ist und DIPMIX wie in Anspruch 13 definiert ist;
und wobei
CAPSSHELL zwei Teile umfasst, wobei die beiden Teile mit Kappe und Körper von CAPSSHELL bezeichnet werden;
die Schritte (1) bis (4) sowohl mit einem Stift, der so geformt ist, dass er die Kappe bereitstellt, als auch mit einem Stift durchgeführt werden, der so geformt ist, dass er den Körper bereitstellt.

17. CAPSSHELL, gefüllt mit einer Formulierung FILLFORM, umfassend einen Wirkstoff ACTINGR, wobei ACTINGR aus der Gruppe ausgewählt sein kann, die aus pharmazeutischem Wirkstoff, Medikament und einer Mischung davon besteht;
wobei CAPSSHELL wie in einem oder mehreren der Ansprüche 1 bis 12 definiert ist.

**18.** Verwendung von CAPSSHELL zum Füllen mit FILLFORM, wobei CAPSSHELL wie in Anspruch 1 definiert ist und FILLFORM wie in Anspruch 17 definiert ist.

**19.** Verwendung von CAPSSHELL in Trockenpulverinhalatoren, wobei CAPSSHELL wie in einem oder mehreren der Ansprüche 1 bis 12 und 17 definiert ist.

**20.** Trockenpulverinhalator mit in den Trockenpulverinhalator eingesetzter CAPSSHELL, wobei CAPSSHELL wie in einem oder mehreren der Ansprüche 1 bis 12 und 17 definiert ist.


**Revendications**

**1.** Enveloppe de capsule CAPSSHELL comprenant HPMC et $CaCl_2$ ;

la quantité de $CaCl_2$ comprise dans CAPSSHELL est de 3 000 à 9 000 ppm sur la base du poids de HPMC compris dans CAPSSHELL ;
CAPSSHELL ne contient pas de gélifiant ; ou
CAPSSHELL ne contient pas de combinaison du gélifiant avec un auxiliaire de gélification,
dans laquelle ledit gélifiant est choisi dans le groupe constitué de : la gomme d'agar, la gomme de guar, la gomme de caroube, le carraghénane, la pectine, le xanthane, la gomme gellane, le mannane de konjac et la gélatine ;
et
dans laquelle ledit auxiliaire de gélification est choisi dans le groupe constitué de : un cation tel que $K^+$, $Na^+$, $Li^+$, $NH4^+$, $Ca^{2+}$ et $Mg^{2+}$.

**2.** CAPSSHELL selon la revendication 1, dans laquelle

HPMC est choisi dans le groupe constitué de
HPMC 2910 contenant environ 7,0 à 12,0 % de groupe hydroxypropoxy et environ 28,0 à 30,0 % de groupe méthoxy,
HPMC 2906 contenant environ 4,0 à 7,5 % de groupe hydroxypropoxy et environ 27,0 à 30,0 % de groupe méthoxy,
HPMC 2208 contenant environ 4,0 à 12,0 % de groupe hydroxypropoxy et environ 19,0 à 24,0 % de groupe méthoxy, et
HPMC 1828 contenant environ 23,0 à 32,0 % de groupe hydroxypropoxy et environ 16,5 à 20,0 % de groupe méthoxy.

**3.** CAPSSHELL selon la revendication 1, dans laquelle
HPMC a une teneur en méthoxy de 27,0 à 30,0 % (p/p).

**4.** CAPSSHELL selon la revendication 1 ou 2, dans laquelle
HPMC a une teneur en hydroxypropoxy de 4,0 à 12,0 % (p/p).

**5.** CAPSSHELL selon une ou plusieurs des revendications 1 à 4, dans laquelle
HPMC est HPMC 2906, HPMC 2910 ou un mélange de ceux-ci.

**6.** CAPSSHELL selon une ou plusieurs des revendications 1 à 5, dans laquelle
CAPSSHELL contient de 3 500 à 9 000 ppm de $CaCl_2$, le ppm étant basé sur le poids de HPMC compris dans CAPSSHELL.

**7.** CAPSSHELL selon une ou plusieurs des revendications 1 à 6, dans laquelle
CAPSSHELL contient de 4 000 à 9 000 ppm de $CaCl_2$, le ppm étant basé sur le poids de HPMC compris dans CAPSSHELL.

8. CAPSSHELL selon une ou plusieurs des revendications 1 à 7, dans laquelle
CAPSSHELL contient 70 % en poids ou plus de HPMC, le % en poids étant basé sur le poids de CAPSSHELL sèche.

9. CAPSSHELL selon une ou plusieurs des revendications 1 à 8, dans laquelle
CAPSSHELL comprend un ou plusieurs additifs ADD, les ADD peuvent être un modificateur de viscosité, un antimousse, un plastifiant, un lubrifiant, un colorant, un solvant, un auxiliaire de solvant, un tensioactif, un dispersant, un solubilisant, un stabilisant, un correctif, un édulcorant, un absorbant, un adsorbant, un adhésif, un antioxydant, un antiseptique, un conservateur, un déshydratant, un arôme, un parfum, un agent d'ajustement du pH, un liant, un humectant, un agent désintégrant, un agent de contrôle de la libération, un acide, un sel ou un mélange de ceux-ci.

10. CAPSSHELL selon une ou plusieurs des revendications 1 à 8, dans laquelle
CAPSSHELL sèche est constituée de HPMC et de $CaCl_2$.

11. CAPSSHELL selon une ou plusieurs des revendications 1 à 8, dans laquelle
CAPSSHELL sèche se compose de 99,4 à 99,35 % en poids de HPMC et de 0,6 à 0,66 % en poids de $CaCl_2$, les quantités de HPMC et de $CaCl_2$ s'additionnant jusqu'à 100 % en poids, le % en poids étant basé sur le poids de la CAPSSHELL sèche.

12. CAPSSHELL selon une ou plusieurs des revendications 1 à 9, dans laquelle
CAPSSHELL sèche se compose de HPMC, $CaCl_2$ et d'un colorant.

13. Procédé de préparation de CAPSSHELL, dans laquelle

CAPSSHELL est formée par un procédé PROCFORMCAPS permettant de former une enveloppe de capsule à partir d'un mélange DIPMIX, DIPMIX est de l'eau contenant $CaCl_2$ et HPMC ;
avec CAPSSHELL selon la revendication 1.

14. Procédé de préparation de CAPSSHELL selon la revendication 13, dans lequel PROCFORMCAPS est un moulage par extrusion, un moulage par injection, un coulage ou un moulage par trempage.

15. Procédé de préparation de CAPSSHELL selon la revendication 13 ou 14, dans lequel PROCFORMCAPS est un moulage par trempage.

16. Procédé de préparation de CAPSSHELL selon la revendication 15, dans lequel

le moulage par trempage comprend les étapes de :

(1) trempage d'une broche de moule pour la première moitié de CAPSSHELL dans DIPMIX ;
(2) retrait de la broche de moule de DIPMIX tout en permettant à un film de se former sur la broche de moule ;
(3) séchage dudit film sur la broche de moule fournissant la première moitié de CPASSHELL ; et
(4) retrait de la moitié de CAPSSHELL de la broche du moule ;

avec CAPSSHELL selon la revendication 1 et DIPMIX selon la revendication 13 ;
et dans lequel
CAPSSHELL comprend deux parties, les deux parties appelées le capuchon et le corps de CAPSSHELL;
les étapes (1) à (4) sont réalisées à la fois avec une broche qui est conformée pour fournir le capuchon, et avec une broche qui est conformée pour fournir le corps.

17. CAPSSHELL remplie d'une formulation FILLFORM comprenant un ingrédient actif ACTINGR, ACTINGR pouvant être choisi dans le groupe constitué d'un ingrédient actif pharmaceutique, d'un médicament, et d'un mélange de ceux-ci ;
avec CAPSSHELL selon une ou plusieurs des revendications 1 à 12.

18. Utilisation de CAPSSHELL pour le remplissage avec FILLFORM, avec CAPSSHELL selon la revendication 1 et FILLFORM selon la revendication 17.

19. Utilisation de CAPSSHELL dans des inhalateurs de poudre sèche, avec CAPSSHELL selon une ou plusieurs des revendications 1 à 12 et 17.

20. Inhalateur de poudre sèche comprenant l'insertion de CAPSSHELL dans l'inhalateur de poudre sèche, avec CAPSSHELL selon une ou plusieurs des revendications 1 à 12 et 17.

**Fig. 1**

50 µm

**Fig. 2**

Fig. 3

EP 4 106 734 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20150231344 A1
- US 5626871 A
- CN 189846851 A
- CN 106166143 B
- US 20190321301 A1
- US 20170087092 A1
- WO 2008050205 A1

### Non-patent literature cited in the description

- *CHEMICAL ABSTRACTS,* 10035-04-8
- **MURPHY, SEAMUS.** UNDERSTANDING THE AFFECT OF DPI DEVICE AND LACTOSE TYPE ON THE OUTPUT FROM A DEVICE. *Journal of Aerosol Medicine and Pulmonary Drug Delivery.,* 2014, vol. 27, A16-A16
- **M. SHERRY KU et al.** Performance qualification of a new hypromellose capsule: Part I. Comparative evaluation of physical, mechanical and processability quality attributes of Vcaps Plus®, Quali-V® and gelatin capsules. *International Journal of Pharmaceutics,* 15 February 2010, vol. 386 (1-2), 30-41